# EUROPÄISCHE PATENTSCHRIFT

(11) **EP 2 545 866 B1**
(45) Veröffentlichungstag und Bekanntmachung des Hinweises auf die Patenterteilung: **18.10.2017**
(21) Anmeldenummer: 12005051.3
(22) Anmeldetag: 07.07.2012
(51) Int. Cl.: A61B 17/32, A61B 17/3211, A61B 17/00

(54) **Medizinisches Schneidinstrument zum Schneiden von Muskeln und Sehnen**
Medical cutting instrument for cutting muscles and tendons
Instrument médical coupant pour couper des muscles et des tendons

(30) Priorität: 13.07.2011 DE 102011107176
(43) Veröffentlichungstag der Anmeldung: 16.01.2013
(73) Patentinhaber: Karl Storz GmbH & Co. KG, 78532 Tuttlingen (DE)
(72) Erfinder: Berberich, Sascha, 78532 Tuttlingen (DE)
(74) Vertreter: Hofmeister, Frank

(56) Entgegenhaltungen:
- DE-U1- 8 421 587
- SU-A1- 1 456 113
- US-A- 253 359

## Beschreibung

Die Erfindung betrifft ein medizinisches Schneidinstrument zum Schneiden von Muskeln und Sehnen, mit einem Schaft, an dessen distalem Ende ein aus zwei Maulteilen bestehendes Werkzeug angeordnet ist, wobei ein Maulteil als gegenüber dem anderen Maulteil verstellbares Maulteil ausgebildet ist, und an dessen proximalem Ende eine Handhabe angeordnet ist, wobei das verstellbare Maulteil und die Handhabe über ein im Schaft gelagertes Betätigungselement derart in Wirkverbindung miteinander stehen, dass das verstellbare Maulteil durch Betätigen der Handhabe zwischen einer geschlossenen Position und einer geöffneten Position des Werkzeugs verstellbar ist.

Gattungsgemäße medizinisches Schneidinstrumente werden beispielsweise zur Entnahme von Sehnen verwendet, um diese als Transplantat zum Ersatz anderer zerstörter Sehnen zu verwenden.

Ein medizinisches Schneidinstrument zum Schneiden von Muskeln und Sehnen ist beispielsweise aus dem US-Patent US 253,359 A bekannt. Mit diesem bekannten Instrument ist es möglich, eine Inzision zu schaffen, die eine Sehne freilegt, diese Sehne zu bergen sowie ein Stück aus dem Sehnengewebe herauszutrennen. Das Gebrauchsmuster DE8421587U offenbart ein medizinisches Schneidinstrument gemäß dem Oberbegriff des Anspruchs 1.

Als Transplantat zum Ersatz des vorderen Kreuzbandes hat sich in der operativen Praxis die Verwendung der Quadrizepssehne bewährt. Bei den bekannten operativen Verfahren zur Entnahme des Quadrizepssehnen-Transplantats wird die Sehne mit einem Skalpell vom Knochen abgetrennt. Nachteilig an diesem bekannten Operationsverfahren zur Entnahme des Quadrizepssehnen-Transplantats ist, dass kein kontrolliertes Durchtrennen der Sehne möglich ist.

Davon ausgehend liegt der Erfindung die **Aufgabe** zugrunde, ein medizinisches Schneidinstrument der eingangs genannten Art zu schaffen, das bei einfacher Handhabung ein kontrolliertes Durchtrennen des Muskel-/Sehnengewebes bei subkutaner Anwendung ermöglicht.

Die **Lösung** dieser Aufgabenstellung ist erfindungsgemäß gekennzeichnet durch die Merkmale des Anspruchs 1. Vorteilhafte Weiterbildungen der Erfindung sind Gegenstand der Unteransprüche.

Die erfindungsgemäße **Lösung** zeichnet sich dadurch aus, dass das andere Maulteil in der Draufsicht U-förmig so ausgebildet ist, dass die beiden parallelen Schenkel des U quer zur Instrumentenlängsachse angeordnet sind und die Öffnung des U zur einer Seite des anderen Maulteils weist, so dass das andere Maulteil von seitlich auf das zu durchtrennende Muskel-/Sehnengewebe aufschiebbar ist.

Durch die erfindungsgemäße Ausbildung des zu einer Seite hin offenen anderen Maulteils ist es möglich, das starre Maulteil kontrolliert subcutan von der Seite auf das zu durchtrennende Muskel-/Sehnengewebe aufzuschieben, um das Muskel-/Sehnegewebe endständig durchtrennen zu können.

Gemäß der Erfindung wird vorgeschlagen, dass zumindest am verstellbaren Maulteil eine Schneidkante ausgebildet ist, die vorzugsweise zum anderen Maulteil hin ausgerichtet am distalen Ende des verstellbaren Maulteils angeordnet ist, so dass das zwischen den beiden Maulteilen angeordnete Muskel-/Sehnengewebe beim Schließen des verstellbaren Maulteils automatisch durchtrennt wird.

Mit einer bevorzugten Ausführungsform der Erfindung wird vorgeschlagen, dass an beiden Maulteilen V-förmig ausgebildete Schneidkanten angeordnet sind. Die V-förmige Ausgestaltung der Schneidkanten bewirkt, dass die zu schneidende Sehne zuerst an ihrer Außenseite fixiert wird, bevor sie dann endgültig im zentralen Bereich abgeschnitten wird. Durch diese äußere Fixierung wird ein deutlich besseres Schneidergebnis erreicht und ein unerwünschtes Quetschen und dadurch mögliches Verschieben der Sehne beim Schneidvorgang verhindert.

Weiterhin wird mit der Erfindung vorgeschlagen, dass die Schneidkante kammartig gezahnt ausgebildet ist, so dass das verstellbare Mauteil kontrolliert auf das zuvor gesplittete Muskel-/Sehnengewebe aufgesetzt werden kann und dieses führend hält, bis das Muskel-/Sehnengewebe beim Schließen der Maulteile durchtrennt wird.

Um das seitliche Aufschieben des starren Maulteils auf das zu durchtrennende Muskel-/Sehnengewebe zu erleichtern, wird erfindungsgemäß vorgeschlagen, dass am freien Ende des distalseitigen Schenkels des U-förmigen anderen Maulteils ein Anlaufschräge ausgebildet ist

Zur Einstellung und Verstellung der Schneidtiefe wird mit der Erfindung vorgeschlagen, dass im Bereich der Handhabe ein Hebelmechanismus vorgesehen ist, der einen variablen Anschlag für das Schließen der beiden Maulteile gegeneinander ausbildet und so die Verstellung der Schneidtiefe bewirkt.

Schließlich wird mit der Erfindung vorgeschlagen, dass an der Handhabe eine Skalierung angeordnet ist, um das Muskel-/Sehnengewebe gezielt in einer bestimmten Tiefe durchtrennen zu können.

Um die Handhabung des erfindungsgemäßen Schneidinstruments zu verbessern und eine sichere Schneidwirkung bzw. Dosierung der Schneidwirkung zu schaffen, wird mit der Erfindung weiterhin vorgeschlagen, dass die Handhabe winklig zur Instrumentenlängsachse am Schaft angeordnet ist. Vorteilhafterweise ist die Handhabe unter einem Winkel von 20° zur Instrumentenlängsachse gegenüber dem Schaft abgewinkelt.

Durch diese Abwinklung ist bei der Entnahme bzw. beim Abschneiden der Quadrizepssehne eine bessere Handhabung und Betätigung des Schneidmechanismus gegeben, da bei einer linear gestreckten Anordnung der Handhabe in Verlängerung des Schaftes der Körper des Patienten die Betätigung des Schneidinstruments behindern kann.

Weitere Merkmale und Vorteile der Erfindung ergeben sich anhand der zugehörigen Zeichnungen, in denen ein Ausführungsbeispiel eines erfindungsgemäßen medizinischen Schneidinstruments zum Schneiden von Muskeln und Sehnen nur beispielhaft dargestellt ist, ohne die Erfindung auf dieses Ausführungsbeispiel zu beschränken. In den Zeichnungen zeigt:
- Fig. 1: eine schematische Seitenansicht eines erfindungsgemäßen medizinischen Schneidinstruments zum Schneiden von Muskeln und Sehnen;
- Fig. 2: eine vergrößerte Ansicht des Details II gemäß Fig. 1, jedoch das Werkzeug im geöffneten Zustand darstellend;
- Fig. 3: eine vergrößerte Draufsicht auf das Detail III gemäß Fig. 1 und
- Fig. 4: eine Ansicht von hinten-unten der Darstellung gemäß Fig. 3.

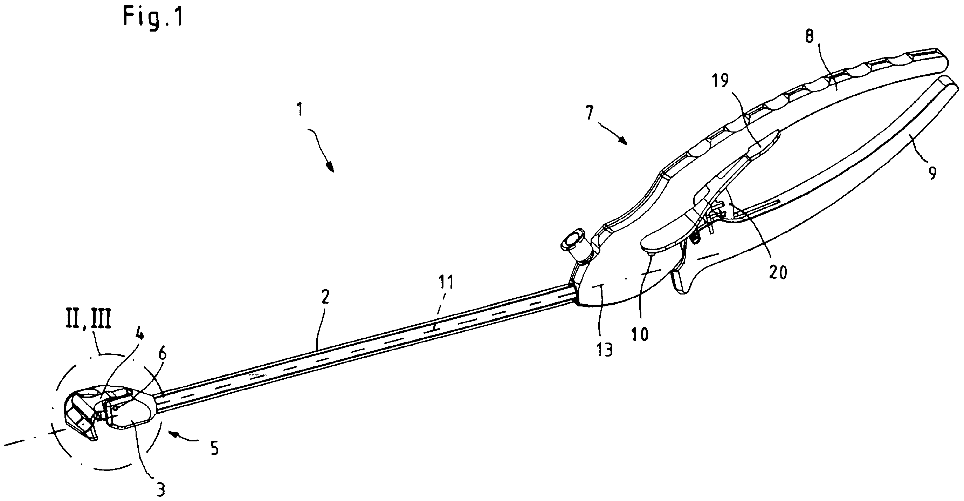

Die Abbildung Fig. 1 zeigt eine perspektivische Ansicht eines medizinisches Schneidinstruments 1 zum Schneiden von Muskeln und Sehnen.

Dieses beispielsweise als Stanze zur Entnahme der Quadrizepssehne ausgebildete Schneidinstrument 1 weist einen Schaft 2 auf, an dessen distalem Ende ein aus zwei Maulteilen 3 und 4 bestehendes Werkzeug 5 angeordnet ist, wobei ein Maulteil 3 starr ausgebildet ist und das andere Maulteil 4 als um eine Schwenkachse 6 gegenüber dem starren Maulteil 3 verschwenkbares Maulteil 4 ausgebildet ist. Am proximalem Ende des Schaftes 2 ist eine Handhabe 7 angeordnet, die bei der dargestellten Ausführungsform aus zwei Griffteilen 8 und 9 besteht, wobei ein Griffteil 8 starr ausgebildet ist und das andere Griffteil 9 als um eine Schwenkachse 10 gegenüber dem starren Griffteil 8 verschwenkbares Griffteil 9 ausgebildet ist.

Wie weiterhin aus Fig. 1 ersichtlich, ist die Handhabe 7 nicht geradlinig in Verlängerung des Schaftes 2 am proximalen Ende des Schaftes 2 angeordnet, sondern vorzugsweise um einen Winkel von 20° abgewinkelt. Durch diese Abwinklung der Handhabe 7 gegenüber dem Schaft 2 ist bei der Entnahme bzw. beim Abschneiden der Quadrizepssehne eine bessere Handhabung und Betätigung des Schneidmechanismus möglich, da bei einer linear gestreckten Anordnung der Handhabe 7 in Verlängerung des Schaftes 2 der Körper des Patienten die Betätigung des Schneidinstruments behindern kann.

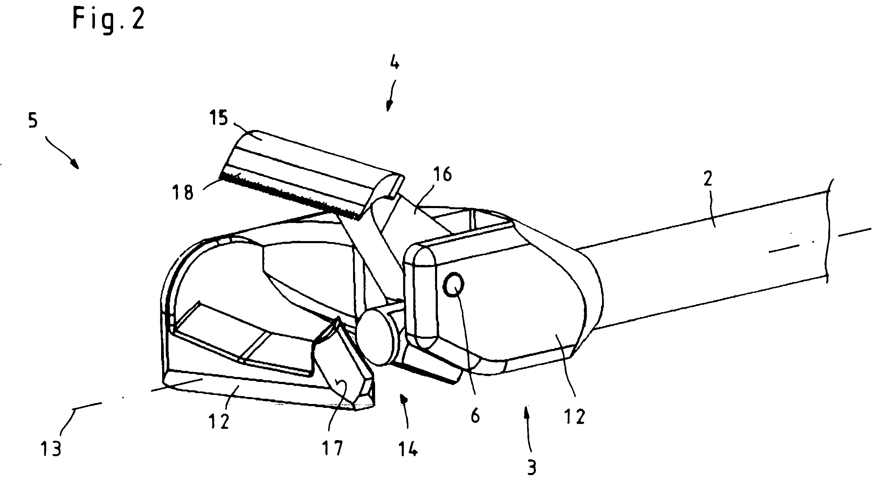

Das verschwenkbare Maulteil 4 des Werkzeugs 5 und das verschwenkbare Griffteil 9 der Handhabe 7 stehen über ein im Schaft 2 gelagertes Betätigungselement 11 derart in Wirkverbindung miteinander, dass das verschwenkbare Maulteil 4 durch Betätigen des verschwenkbaren Griffteils 9 zwischen einer am starren Maulteil 3 anliegenden geschlossenen Position (Fig. 1, 3 und 4) und einer gegenüber dem starren Maulteil 3 verschwenkten offenen Position (Fig. 2) verstellbar ist. Durch das starre Maulteil 3 kann eine besonders vorteilhafte Führung und damit Positionierung der Sehne beim Schnitt erreicht werden, was bei der subkutanen Anwendung des Schneidinstrumentes 1 zu einem wesentlich besseren Schneidergebnis führt. Alternativ zu der dargestellten Ausführungsform des Schneidinstruments 1 mit einem starren Maulteil 3 und einem verschwenkbaren Maulteil 4 ist es selbstverständlich auch möglich aber nicht gemäß der Erfindung, beide Maulteile verschwenkbar auszugestalten. Gerade für freischwebende, freipräparierbare Sehnen erweist sich eine Variante mit zwei verschwenkbaren Maulteilen als besonders sinnvoll.

Weiterhin ist das dargestellte Verschwenken des Maulteils 4 gegenüber dem anderen Maulteil 3 eine Ausführungsform gemäß der Erfindung zum gegenseitigen Verstellen der Maulteile 3 und 4 zueinander. Neben dem Verschwenken um die Schwenkachse 6 ist es beispielsweise auch möglich, die Maulteile 3 und 4 alternativ aber nicht gemäß der Erfindung oder zusätzlich axial gegeneinander zu verschieben.

Der Aufbau des aus den Mauteilen 3 und 4 bestehenden distalseitigen Werkzeugs 5 ist insbesondere den vergrößerten Detailansichten der Abbildungen Fig. 2 bis 4 zu entnehmen.

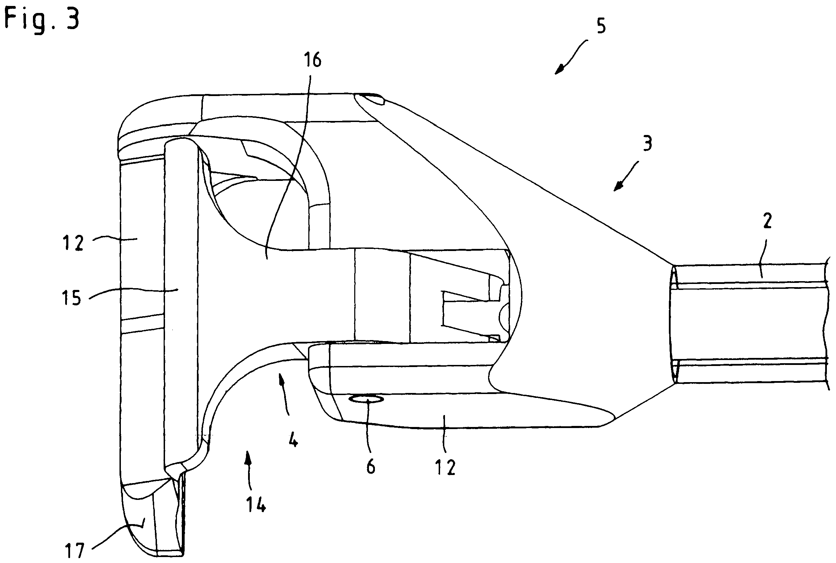

Wie insbesondere aus den Abbildungen Fig. 2 und 3 ersichtlich, ist das starre Maulteil 3 in der Draufsicht U-förmig so ausgebildet ist, dass die beiden parallelen Schenkel 12 des U quer zur Instrumentenlängsachse 13 angeordnet sind und die Öffnung 14 des U zur einer Seite des starren Maulteils 3 weist.

Das verschwenkbare Maulteil 4 ist aus der Draufsicht T-förmig so ausgebildet, dass der Quersteg 15 des T parallel zum distalseitigen Schenkel 12 des U-förmigen starren Mauteils 3 angeordnet ist und der Längssteg 16 in Verlängerung des Betätigungselements 11 angeordnet ist.

Durch die Ausbildung des starren Maulteils 3 als zu einer Seite hin offenes Maulteil 3 und durch die T-förmige Ausbildung des verschwenkbaren Maulteils 4 ist es möglich, das Werkzeug 5 von seitlich auf das zu durchtrennende Muskel-/Sehnengewebe aufzuschieben, um so ein kontrolliertes endständiges Durchtrennen des Muskel-/Sehnengewebes zu ermöglichen.

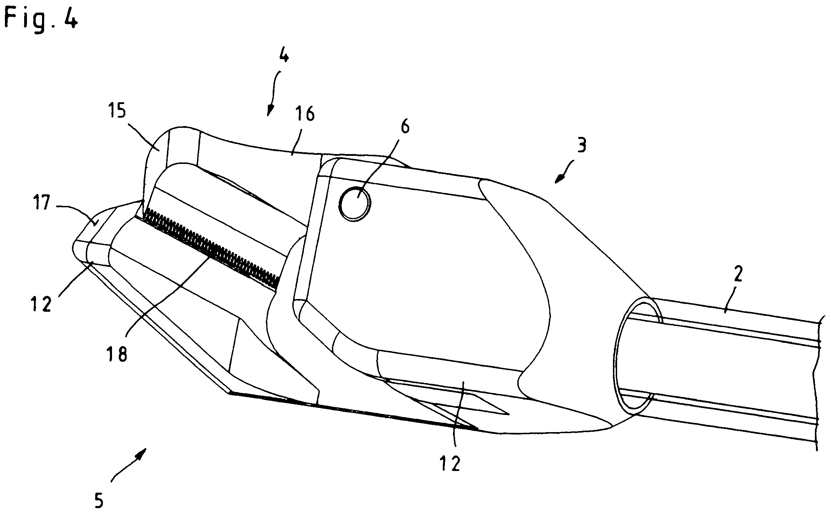

Um das seitliche Aufschieben des starren Maulteils 3 auf das zu durchtrennende Muskel-/Sehnengewebe zu erleichtern, ist am freien Ende des distalseitigen Schenkels 12 des U-förmigen starren Maulteils 3 ein Anlaufschräge 17 ausgebildet, wie dies der Abbildung Fig. 4 zu entnehmen ist.

Zum Durchtrennen des Muskel-/Sehnengewebes ist am verschwenkbaren Maulteil 4 eine Schneidkante 18 angeordnet, die nach unten zum starren Maulteil 3 hin ausgerichtet so am distalseitigen Quersteg 15 des verschwenkbaren Maulteils 4 angeordnet, dass das zwischen den Maulteilen 3 und 4 angeordnete Muskel-/Sehnengewebe durchtrennt wird, sobald das verschwenkbare Maulteil 4 des Werkzeugs 5 vollständig geschlossen wird.

Alternativ zur Ausbildung der Schneidkante 18 nur am verschwenkbaren Maulteil 4 ist es selbstverständlich auch möglich, sowohl am verschwenkbaren Maulteil 4 als auch am starren Maulteil 3 Schneidkanten 18, vorzugsweise V-förmige Schneidkanten 18, vorzusehen.

Wie aus Fig. 4 ersichtlich, ist die Schneidkante 18 kammartig gezahnt ausgebildet, so dass das verschwenkbare Mauteil 4 kontrolliert auf das zuvor gesplittete Muskel-/Sehnengewebe aufgesetzt werden kann und dieses führend hält, bis das Muskel-/Sehnengewebe beim Schließen der Maulteile 3 und 4 durchtrennt wird.

Wie aus Fig. 1 ersichtlich, ist zur Einstellung und Verstellung der Schneidtiefe an der Handhabe 7 ein Hebelmechanismus 19 vorgesehen, der einen variablen Anschlag für das Schließen der beiden Maulteile 3 und 4 gegeneinander ausbildet und so die Verstellung der Schneidtiefe bewirkt.

Weiterhin ist an der Handhabe 7 eine von außen ablesbare Skalierung 20 vorgesehen, damit das Muskel-/Sehnengewebe durch die Betätigung des Hebelmechanismus 19 gezielt in einer bestimmten Tiefe durchtrennt werden kann. Dieser Hebelmechanismus 19 mit der Skalierung 20 zusammen mit der kammartig gezahnten Schneidkante 18 erlaubt eine äußerst präzise Einstellung der Schnitttiefe bei gleichzeitiger exakter Platzierung des durchzuführenden Schnitts.

Durch die voranstehend beschriebene Ausbildung der Maulteile 3 und 4 des medizinischen Schneidwerkzeugs 1 als seitlich offenes Werkzeug 5 ermöglicht es, das starre Maulteil 3 kontrolliert subcutan von der Seite auf das zu durchtrennende Muskel-/Sehnengewebe aufzuschieben, um das Muskel-/Sehnegewebe endständig durchtrennen zu können.

**Bezugszeichenliste**

| | | | |
|---|---|---|---|
| 1 | medizinisches Schneidinstrument | 18 | Schneidkante |
| 2 | Schaft | 19 | Hebelmechanismus |
| 3 | anderes / starres Maulteil | 20 | Skalierung |
| 4 | verstellbares / verschwenkbares Maulteil | | |
| 5 | Werkzeug | | |
| 6 | Schwenkachse | | |
| 7 | Handhabe | | |
| 8 | starres Griffteil | | |
| 9 | verschwenkbares Griffteil | | |
| 10 | Schwenkachse | | |
| 11 | Betätigungselement | | |
| 12 | Schenkel | | |
| 13 | Instrumentenlängsachse | | |
| 14 | Öffnung | | |
| 15 | Quersteg | | |
| 16 | Längssteg | | |
| 17 | Anlaufschräge | | |

## Patentansprüche

1. Medizinisches Schneidinstrument zum Schneiden von Muskeln und Sehnen, mit einem Schaft (2), an dessen distalem Ende ein aus zwei Maulteilen (3, 4) bestehendes Werkzeug (5) angeordnet ist, wobei ein Maulteil (4) als gegenüber dem anderen Maulteil (3) verstellbares Maulteil (4) ausgebildet ist, und an dessen proximalem Ende eine Handhabe (7) angeordnet ist, wobei das verstellbare Maulteil (4) und die Handhabe (7) über ein im Schaft (2) gelagertes Betätigungselement (11) derart in Wirkverbindung miteinander stehen, dass das verstellbare Maulteil (4) durch Betätigen der Handhabe (7) zwischen einer geschlossenen Position und einer geöffneten Position des Werkzeugs (5) verstellbar ist,
**dadurch gekennzeichnet,**
**dass** ein Maulteil (3) starr ausgebildet ist und das andere Maulteil (4) als um eine Schwenkachse (6) gegenüber dem starren Maulteil (3) verschwenkbares Maulteil (4) ausgebildet ist, wobei das starre Maulteil (3) in der Draufsicht U-förmig so ausgebildet ist, dass die beiden parallelen Schenkel (12) des U quer zur Instrumentenlängsachse (13) angeordnet sind und die Öffnung (14) des U zur einer Seite des starren Maulteils (3) weist, so dass das starre Maulteil (3) von seitlich auf das zu durchtrennende Muskel-/Sehnengewebe aufschiebbar ist und, dass das verschwenkbare Maulteil (4) aus der Draufsicht T-förmig so ausgebildet ist, dass der Quersteg (15) des T parallel zum distalseitigen Schenkel (12) des U-förmigen starren Maulteils (3) angeordnet ist und der Längssteg (16) in Verlängerung des Betätigungselements (11) angeordnet ist, wobei zumindest am distalseitigen Quersteg (15) des verschwenkbaren Maulteils (4) eine Schneidkante (18) ausgebildet ist, die zum starren Maulteil (3) hin ausgerichtet ist.

2. Medizinisches Schneidinstrument nach Anspruch 1, **dadurch gekennzeichnet, dass** an beiden Maulteilen (3 und 4) V-förmig ausgebildete Schneidkanten (18) angeordnet sind.

3. Medizinisches Schneidinstrument nach Anspruch 1 oder 2, **dadurch gekennzeichnet, dass** die Schneidkante (18) kammartig gezahnt ausgebildet ist.

4. Medizinisches Schneidinstrument nach einem der Ansprüche 1 bis 3, **dadurch gekennzeichnet, dass** am freien Ende des distalseitigen Schenkels (12) des U-förmigen starren Maulteils (3) ein Anlaufschräge (17) ausgebildet ist.

5. Medizinisches Schneidinstrument nach einem der Ansprüche 1 bis 4, **dadurch gekennzeichnet, dass** im Bereich der Handhabe (7) ein Hebelmechanismus (19) angeordnet ist, der einen variablen Anschlag für das Schließen der Maulteile (3 und 4) gegeneinander bildet und so eine Verstellung der Schneidtiefe bewirkt.

6. Medizinisches Schneidinstrument nach Anspruch 5, **dadurch gekennzeichnet, dass** an der Handhabe (7) eine Skalierung (20) zur Bestimmung der Schnitttiefe angeordnet ist.

7. Medizinisches Schneidinstrument nach einem der Ansprüche 1 bis 6, **dadurch gekennzeichnet, dass** die Handhabe (7) winklig zur Instrumentenlängsachse (13) am Schaft (2) angeordnet ist.

8. Medizinisches Schneidinstrument nach Anspruch 7, **dadurch gekennzeichnet, dass** die Handhabe (7) unter einem Winkel von insbesondere 20° zur Instrumentenlängsachse (13) am Schaft (2) angeordnet ist.

## Claims

1. A medical cutting instrument for cutting muscles and tendons, having a shaft (2) on whose distal end a tool (5) is mounted that consists of two jaw members (3, 4), such that one jaw member (4) is configured as a jaw member (4) that can be moved with respect to the other jaw member (3), and on whose proximal end a handle is mounted (7), such that the movable jaw member (4) and the handle (7) operatively interact with one another by way of an actuation element (11) mounted in the shaft (2), in such a way that the movable jaw member (4) can be shifted by actuation of the handle (7) between a closed position and an opened position of the tool (5),
**characterised in that**
one jaw member (3) is configured rigidly and the other jaw member (4) is configured as a jaw member (4) that is pivotable about a pivot axis (6) relative to the rigid jaw member (3), wherein the rigid jaw member (3) seen from overhead is U-shaped in such a way that the two parallel legs (12) of the U are disposed diagonally to the instrument longitudinal axis (13) and the opening (14) of the U points to one side of the rigid jaw member (3), so that the rigid jaw member (3) can be pushed from the side onto the muscle/tendon tissue that is to be severed, and that the pivotable jaw member (4) seen from overhead is T-shaped in such a way that the crossbar (15) of the T is disposed parallel to the distal-side leg (12) of the U-shaped rigid jaw member (3) and the longitudinal bar (16) is disposed as extension of the actuation element (11), wherein at least at the distal-side crossbar (15) of the pivotable jaw member (4) a cutting edge (18) is configured which is oriented toward the rigid jaw member (3).

2. The medical cutting instrument according to claim 1, **characterised in that** V-shaped cutting edges (18) are mounted on both jaw members (3 and 4).

3. The medical cutting instrument according to claim 1 or 2, **characterised in that** the cutting edge (18) is of toothed configuration in the manner of a comb.

4. The medical cutting instrument according to any one of claims 1 to 3, **characterised in that** a run-up slope (17) is configured on the free end of the distal-side leg (12) of the U-shaped rigid jaw member (3).

5. The medical cutting instrument according to any one of claims 1 to 4, wherein there is mounted in the area of the handle (7) a lever mechanism (19) that constitutes a variable stop for closing the jaw members (3 and 4) with respect to one another and thus causes a shifting of the cutting depth.

6. The medical cutting instrument according to claim 5, **characterised in that** a scale (20) is mounted on the handle (7) to determine the cutting depth.

7. The medical cutting instrument according to any one of claims 1 to 6, **characterised in that** the handle (7) is disposed on the shaft (2) at an angle to the instrument longitudinal axis (13).

8. The medical cutting instrument according to claim 7, **characterised in that** the handle (7) is disposed on the shaft (2) at an angle, in particular of 20°, to the instrument longitudinal axis (13).

## Revendications

1. Instrument de coupe médical destiné à couper des muscles et des tendons, comprenant un corps allongé (2) à l'extrémité distale duquel est agencé un outil (5) constitué de deux pièces de mâchoire (3, 4), une pièce de mâchoire (4) étant réalisée en tant que pièce de mâchoire (4) pouvant être déplacée par rapport à l'autre pièce de mâchoire (3), et à l'extrémité proximale duquel est agencée une poignée de manoeuvre (7), la pièce de mâchoire (4) déplaçable et la poignée de manoeuvre (7) étant en liaison interactive par l'intermédiaire d'un élément d'actionnement (11) monté dans le corps allongé (2) de façon telle, que la pièce de mâchoire (4) déplaçable puisse être déplacée par l'actionnement de la poignée de manoeuvre (7), entre une position fermée et une position ouverte de l'outil (5),
**caractérisé**
**en ce qu'**une pièce de mâchoire (3) est de configuration fixe et l'autre pièce de mâchoire (4) est réalisée sous forme de pièce de mâchoire (4) pouvant pivoter autour d'un axe de pivotement (6) par rapport à la pièce de mâchoire (3) fixe, la pièce de mâchoire (3) fixe présentant en vue de dessus, une configuration en forme de U de manière telle, que les deux ailes parallèles (12) du U soient agencées transversalement à l'axe longitudinal d'instrument (13), et l'ouverture (14) du U soit dirigée vers un côté de la pièce de mâchoire (3) fixe de façon à ce que la pièce de mâchoire (3) fixe puisse être engagée latéralement sur des tissus de muscles ou de tendons, et **en ce que** la pièce de mâchoire (4) pivotante présente en vue de dessus, une configuration en forme de T de manière telle, que la branche transversale (15) du T soit agencée parallèlement à l'aile (12) côté distal de la pièce de mâchoire (3) fixe en forme de U, et que la branche longitudinale (16) soit agencée en prolongement de l'élément d'actionnement (11), une arête de coupe (18) étant formée au moins sur la branche transversale (15) du côté distal de la pièce de mâchoire (4) pivotante, et étant dirigée vers la pièce de mâchoire (3) fixe.

2. Instrument de coupe médical selon la revendication 1, **caractérisé en ce que** sur les deux pièces de mâchoire (3 et 4) sont agencées des arêtes de coupe (18) de configuration en forme de V.

3. Instrument de coupe médical selon la revendication 1 ou la revendication 2, **caractérisé en ce que** l'arête de coupe (18) est d'une configuration dentée en forme de peigne.

4. Instrument de coupe médical selon l'une des revendications 1 à 3, **caractérisé en ce qu'**à l'extrémité libre de l'aile (12) côté distal de la pièce de mâchoire (3) fixe en forme de U, est formée une rampe d'engagement (17).

5. Instrument de coupe médical selon l'une des revendications 1 à 4, **caractérisé en ce que** dans la zone de la poignée de manoeuvre (7) est agencé un mécanisme à levier (19), qui forme une butée variable pour la fermeture des pièces de mâchoire (3 et 4) l'une vers l'autre et produit ainsi un réglage de la profondeur de coupe.

6. Instrument de coupe médical selon la revendication 5, **caractérisé en ce que** sur la poignée de manoeuvre (7) est agencée une graduation (20) pour déterminer la profondeur de coupe.

7. Instrument de coupe médical selon l'une des revendications 1 à 6, **caractérisé en ce que** la poignée de manoeuvre (7) est agencée angulairement par rapport à l'axe longitudinal d'instrument (13), sur le corps allongé (2).

8. Instrument de coupe médical selon la revendication 7, **caractérisé en ce que** la poignée de manoeuvre (7) est agencée sous un angle de notamment 20° par rapport à l'axe longitudinal d'instrument (13), sur le corps allongé (2).
